# EUROPEAN PATENT APPLICATION

(11) **EP 4 710 775 A1**
(43) Date of publication of application: **18.03.2026**
(21) Application number: 24807147.4
(22) Date of filing: 10.05.2024
(51) Int. Cl.: A23L 33/13, A23C 9/152, A23C 9/154, A61K 31/706, A61K 45/00, A61P 3/02, A61P 3/06, A61P 3/10, A61P 21/00

(54) **COMPOSITION FOR PROMOTING CHILD DEVELOPMENT**

(30) Priority: 12.05.2023 JP 2023079513; 12.05.2023 JP 2023079514; 12.05.2023 JP 2023079515; 12.05.2023 JP 2023079516
(71) Applicant: Meiji Holdings Co., Ltd., Tokyo 104-0031 (JP)
(72) Inventor: WAKABAYASHI, Jun, Hachioji-shi, Tokyo 192-0919 (JP); HATTORI, Kouya, Hachioji-shi, Tokyo 192-0919 (JP); MORIFUJI, Masashi, Hachioji-shi, Tokyo 192-0919 (JP)
(74) Representative: TBK
(86) International application number: PCT/JP2024/017394
(87) International publication number: WO 2024/237195

(57) **Abstract**

An object of the present invention is to clarify a physiological significance or function such as an influence of nicotinamide mononucleotide (NMN) contained in breast milk on growth of a child. Provided is a composition for improving exercise capacity of children, comprising a precursor of NAD. The improvement in exercise capacity is preferably any one selected from the group consisting of promotion of skeletal muscle development and improvement in activity. Provided is a composition for reducing blood triglyceride levels in children, comprising a precursor of NAD. Provided is a composition for reducing blood glucose levels in children, comprising a precursor of NAD. Provided is a composition for inhibiting fat accumulation in children, comprising a precursor of NAD.

## Description

### TECHNICAL FIELD

The present invention relates to a composition for improving exercise capacity of children, a composition for reducing blood triglyceride levels in children, a composition for reducing blood glucose levels in children, and a composition for inhibiting fat accumulation in children. The present invention is useful in the fields of development, health, and nutrition of children, particularly infants and toddlers, and the field of production of foods and beverages for them, and the like.

### BACKGROUND ART

β-Nicotinamide Mononucleotide (hereinafter, referred to as NMN) is a precursor of Nicotinamide Adenine Dinucleotide (hereinafter, referred to as NAD) necessary for human to maintain a vital function, and is a compound belonging to vitamin B3. With regard to NAD, a useful action has been reported so far mainly in relation to aging.

With regard to exercise capacity, for example, it has been reported that in aged mice in which SIRT1, which plays an important role in controlling metabolic reactions in various tissues, is highly expressed in the brain due to NAD-dependent enzyme activity, skeletal muscle function is improved and the amount of voluntary activity is increased (Non Patent Document 1). In addition, it has been reported that a significant improvement in walking speed and left grip strength performance was observed in elderly men to whom 250 mg of NMN was administered per day for 6 weeks or 12 weeks (Non Patent Document 2). Patent Document 1 describes a composition for preventing chemotherapy-induced neurotoxic damage in a subject, the composition containing a compound that increases the level of NAD+ in the subject, wherein the chemotherapy-induced neurotoxic damage includes chemotherapy-induced cognitive impairment (CICI), wherein the subject is i) a subject exposed to a chemotherapy agent, wherein the CICI has not yet been induced, or ii) a subject exposed to a chemotherapy agent, or iii) a subject who is going to be exposed to a chemotherapy agent, wherein the compound that increases the level of NAD+ is an NAD+ precursor selected from a nicotinamide mononucleotide (NMN), nicotinic acid, nicotinamide, nicotinamide riboside (NR), nicotinic acid mononucleotide, or nicotinic acid riboside, or salts thereof. As another embodiment, Patent Document 1 described above describes a method for improving nerve function and motor skills in a subject, including a step of administering to the subject an effective amount of an agent that increases the level of NAD+ in the subject. Patent Document 2 describes a composition for imparting a health benefit to a female mammal and/or a child of the female mammal, containing a specific nicotinamide adenine dinucleotide (NAD) precursor, characterized in that the composition is administered to the female mammal orally, transmucosally (for example, transnasally), by inhalation, by injection, or topically (for example, transdermally), wherein the child orally takes breast milk from the female mammal, thereby imparting the health benefit to the child, and the health benefit imparted to the female mammal is a reduction in weight gain associated with pregnancy, an promotion in weight loss after pregnancy and/or an increase in breast milk production during lactation, and the health benefit imparted to the child is an increase in brain mass, an improvement in physical abilities, and prevention of body weight gain and/or reduction of anxiety due to aging. Patent Document 3 describes a method for increasing exercise capacity of a subject, including a step of administering an effective amount of an NAD+ precursor to the subject.

In addition, with regard to triglycerides, for example, it has been reported that in a test in which NMN is administered to mice after feeding a high-fat diet, lipid metabolism is improved, and the amount of triglycerides in the liver is reduced (Non Patent Document 3). Patent Document 4 describes that when a hard capsule containing 20 mg of resveratrol and 20 mg of β-nicotinamide mononucleotide and a hard capsule containing 20 mg of resveratrol, 20 mg of β-nicotinamide mononucleotide, and 120 mg of niacinamide were taken by three subjects (50's) with a high blood neutral fat value continuously for 4 weeks (28 days), two capsules were taken once a day, the neutral fat value was decreased. Patent Document 5 describes an adiponectin secretion enhancer characterized by containing β-nicotinamide mononucleotide or a pharmacologically acceptable salt thereof and a solvate thereof as active ingredients, and it is stated that the promotion of the secretion of adiponectin makes it possible to treat or prevent systemic metabolic abnormalities associated with obesity, particularly insulin resistance in multiple organs. Patent Document 6 describes a method for treating or preventing at least one physical condition selected from the group consisting of oxidative stress, a condition associated with oxidative stress, a decrease in glutathione levels, and a condition associated with a decrease in glutathione levels, the method including administering an effective amount of a combination of a nicotinamide adenine dinucleotide (NAD+) precursor and at least one ketone or ketone precursor to an individual in need of the combination, and an increase in triglyceride levels is cited as an example of the physical condition.

Further, with regard to the blood glucose levels, for example, it has been reported that impaired glucose tolerance due to feeding of maternal and long term post-weaning high-fat diet of a mouse was improved by administration of NMN (Non Patent Document 3 described above), and that administration of NMN restored intestinal NAD+ levels and obesity-associated metabolic abnormalities, manifested by a decrease in ileal proglucagon expression and GLP-1 production as well as postprandial hyperglycemia in model mice that displayed diminished glucagon-like peptide-1 (GLP-1) production and diet-induced obese mice (Non Patent Document 4). Patent Document 7 describes a glucose uptake capacity increasing agent in muscle tissue containing β-NMN-containing yeast extract as an active ingredient. In addition, Patent Document 8 describes a pharmaceutical composition or a health food composition preferably containing nicotinamide mononucleotide and mogroside, and it is stated that this composition is for reducing blood glucose levels, promoting the growth and repair of islet cells, and hyperglycemia control and/or diabetes control.

Furthermore, with regard to inhibition of fat accumulation, for example, it has been reported that impaired glucose tolerance due to feeding of maternal and long term post-weaning high-fat diet of a mouse was improved by administration of NMN (Non Patent Document 3 described above), and that when NMN was similarly administered to a mouse fed with a high-fat diet, lipid metabolism and obesity-associated metabolic abnormalities indicated by impaired glucose tolerance were improved (Non Patent Document 4 described above).

Patent Document 7 described above describes a glucose uptake capacity increasing agent in muscle tissue containing β-NMN-containing yeast extract as an active ingredient, and it is stated that this agent can be used as a prophylactic or ameliorating agent for various complications such as diabetes and obesity caused by diabetes. In addition, Patent Document 8 described above describes a pharmaceutical composition or a health food composition preferably containing nicotinamide mononucleotide and mogroside, and it is stated that this composition can be used for controlling fat accumulation and body weight.

On the other hand, it has been reported that in the mammary gland of rats, the synthesis of NMN is enhanced specifically in the lactating period (Non Patent Document 5). In addition, human breast milk contains NAD+ metabolites including nicotinamide, and it has recently been revealed that human breast milk particularly contains a large amount of NMN among them (Non Patent Document 6). Further, it has been reported that when using an animal model, nicotinamide riboside (NR) was orally administered to male mice during the lactation period daily from days 2 to 20 after birth and the mice were weaned on day 21 and were assigned to high-fat diet on day 90 for 10 weeks, the mice administered with NR had beneficial effects on lipid and energy metabolism in skeletal muscle and liver in adulthood, as compared with the control group (Non Patent Document 7).

### PRIOR ART REFERENCES

### Patent Documents

Patent Document 1: WO 2016/176437 A (JP 2018-514548 A, JP 7193232 B2)
Patent Document 2: WO 2018/102426 A (JP 2020-504168 A, JP 7136795 B2)
Patent Document 3: WO 2018/112541 A (JP 2020-504722 A)
Patent Document 4: WO 2016/158212 A (JP 6545256 B2)
Patent Document 5: JP 2018-131418 A
Patent Document 6: WO 2019/242982 A (JP 2021-528372 A)
Patent Document 7: JP 2018-203691 A (JP 7093161 B2)
Patent Document 8: WO 2020/177153 A (JP 2022-534165 A)

### Non Patent Documents

Non Patent Document 1: Satoh A et al, Cell Metab. 2013 Sep 3; 18(3): 416-30.
Non Patent Document 2: Igarashi M et al, NPJ Aging. 2022 May 1; 8(1): 5.
Non Patent Document 3: Uddin GM et al, Cells. 2020 Mar 25; 9(4): 791.
Non Patent Document 4: Nagahisa T et al, Endocrinology. 2022 Apr 1; 163(4): bqac023.
Non Patent Document 5: A. L. Greenbaum et al, Biochem J 1 March 1968; 107(1): 55-62.
Non Patent Document 6: J Chromato B 2019; 1110-1111: 74-80
Non Patent Document 7: Alba Serrano et ai, Nutrients 2022, 14, 2259.

### SUMMARY OF THE INVENTION

### Object to be Achieved by the Invention

Although breast milk comprises NMN, the physiological significance and function of NMN, such as its influence on child development, have not been clarified.

### Means for Achieving the Object

The present invention provides the following items.
[1] A children's nutritional composition, comprising a precursor of nicotinamide adenine dinucleotide (NAD), for promotion of child development in any one selected from improvement in exercise capacity, reduction in blood triglycerides, reduction in blood glucose levels, and inhibition of fat accumulation.
[2] The composition according to 1, wherein the composition is a composition for improving exercise capacity, and the improvement in exercise capacity is any one selected from the group consisting of promotion of skeletal muscle development and improvement in activity.
[3] The composition according to 1 or 2, wherein the composition is a composition for improving exercise capacity, and the improvement in exercise capacity is measured by any one selected from the group consisting of improvement in muscle strength, increase in the amount of voluntary activity, and increase in moving speed.
[4] The composition according to any one of 1 to 3, wherein the promotion of child development is promotion of child development after weaning.
[5] The composition according to any one of 1 to 4, wherein the promotion of child development is promotion of child development after 3 years old.
[6] The composition according to any one of 1 to 5, which is to be taken by an infant or toddler.
[7] The composition according to any one of 1 to 5, which is to be taken by a child before weaning.
[8] The composition according to any one of 1 to 7, wherein the composition is in any form selected from the group consisting of a modified milk powder, modified liquid milk, and a breast milk substitute food.
[9] The composition according to any one of 1 to 8, wherein the precursor of NAD is nicotinamide mononucleotide (NMN).
[10] The composition according to any one of 1 to 9, wherein the concentration of NMN during intake is 0.05 µg/mL or more.
[11] The composition according to any one of 1 to 10, which is in a form of a modified milk powder and comprises 0.1 mg or more of NMN per 100 g.
[12] A composition comprising a precursor of nicotinamide adenine dinucleotide (NAD), which is to be taken by a child before weaning for promoting child development after weaning.
[13] The composition according to 12, wherein the promotion of child development is any one selected from improvement in exercise capacity, reduction in blood triglycerides, reduction in blood glucose levels, and inhibition of fat accumulation.

The present invention provides the following items.
[101] A children's nutritional composition comprising a precursor of nicotinamide adenine dinucleotide (NAD) for use in a method for promoting child development in any one selected from improvement in exercise capacity, reduction in blood triglycerides, reduction in blood glucose levels, and inhibition of fat accumulation. Use of a precursor of nicotinamide adenine dinucleotide (NAD) in production of a children's nutritional composition for promotion of child development in any one selected from improvement in exercise capacity, reduction in blood triglycerides, reduction in blood glucose levels, and inhibition of fat accumulation. A method or non-therapeutic method for promotion of child development in any one selected from improvement in exercise capacity, reduction in blood triglycerides, reduction in blood glucose levels, and inhibition of fat accumulation, including a step of administering to a subject a children's nutritional composition comprising a precursor of nicotinamide adenine dinucleotide (NAD). Use or non-therapeutic use of a children's nutritional composition comprising a precursor of nicotinamide adenine dinucleotide (NAD) for promotion of child development in any one selected from improvement in exercise capacity, reduction in blood triglycerides, reduction in blood glucose levels, and inhibition of fat accumulation in a subject.
[102] The composition, use in production, method or non-therapeutic method, and use or non-therapeutic use according to 101 for improving exercise capacity, wherein the improvement in exercise capacity is any one selected from the group consisting of promotion of skeletal muscle development and improvement in activity.
[103] The composition, use in production, method or non-therapeutic method, and use or non-therapeutic use according to 101 or 102 for improving exercise capacity, wherein the improvement in exercise capacity is measured by any one selected from the group consisting of improvement in muscle strength, increase in the amount of voluntary activity, and increase in moving speed.
[104] The composition, use in production, method or non-therapeutic method, and use or non-therapeutic use according to any one of 101 to 103, wherein the promotion of child development is promotion of child development after weaning.
[105] The composition, use in production, method or non-therapeutic method, and use or non-therapeutic use according to any one of 101 to 104, wherein the promotion of child development is promotion of child development after 3 years old.
[106] The composition, use in production, method or non-therapeutic method, and use or non-therapeutic use according to any one of 101 to 105, wherein the composition is to be taken by an infant or toddler.
[107] The composition, use in production, method or non-therapeutic method, and use or non-therapeutic use according to any one of 101 to 105, wherein the composition is to be taken by a child before weaning.
[108] The composition, use in production, method or non-therapeutic method, or use or non-therapeutic use according to any one of 101 to 107, wherein the composition is in any form selected from the group consisting of a modified milk powder, modified liquid milk, and a breast milk substitute food.
[109] The composition, use in production, method or non-therapeutic method, and use or non-therapeutic use according to any one of 101 to 108, wherein the precursor of NAD is nicotinamide mononucleotide (NMN).
[110] The composition, use in production, method or non-therapeutic method, and use or non-therapeutic use according to any one of 101 to 109, wherein the concentration of NMN during intake is 0.05 µg/mL or more.
[111] The composition, use in production, method or non-therapeutic method, and use or non-therapeutic use according to any one of 101 to 110, wherein the composition is in a form of a modified milk powder and comprises 0.1 mg or more of NMN per 100 g.
[112] A precursor of nicotinamide adenine dinucleotide (NAD) or a composition comprising the same, for use in a method in which the precursor or the composition comprising the same is taken by a child before weaning to promote child development after weaning. Use of a composition for use in a method in which a precursor of nicotinamide adenine dinucleotide (NAD) is taken by a child before weaning to promote child development after weaning. A method or a non-therapeutic method in which a precursor of nicotinamide adenine dinucleotide (NAD) is taken by a child before weaning to promote child development after weaning, including a step of administering the precursor to a subject. Use or non-therapeutic use in which a precursor of nicotinamide adenine dinucleotide (NAD) is taken by a child before weaning for promoting child development after weaning.
[113] The composition, use in production, method or non-therapeutic method, and use or non-therapeutic use according to 112, wherein the promotion of child development is any one selected from improvement in exercise capacity, reduction in blood triglycerides, reduction in blood glucose levels, and inhibition of fat accumulation.

The present invention provides the following items.
[201] A composition for improving exercise capacity of children, comprising a precursor of nicotinamide adenine dinucleotide (NAD).
[202] The composition according to 201, wherein the improvement in exercise capacity is any one selected from the group consisting of promotion of skeletal muscle development and improvement in activity.
[203] The composition according to 201 or 202, wherein the improvement in exercise capacity is measured by any one selected from the group consisting of improvement in muscle strength, increase in the amount of voluntary activity, and increase in moving speed.
[204] The composition according to any one of 201 to 203, wherein the improvement in exercise capacity of children is an improvement in exercise capacity of infants or toddlers.
[205] The composition according to any one of 201 to 204, wherein the improvement in exercise capacity of children is an improvement in exercise capacity of children after weaning.
[206] The composition according to any one of 201 to 205, which is to be taken by an infant or toddler.
[207] The composition according to any one of 201 to 205, which is to be taken by a child before weaning.
[208] The composition according to any one of 201 to 207, wherein the composition is in any form selected from the group consisting of a modified milk powder, modified liquid milk, and a breast milk substitute food.
[209] The composition according to any one of 201 to 208, wherein the precursor of NAD is nicotinamide mononucleotide (NMN).
[210] The composition according to 209, wherein the concentration of NMN during intake is 0.05 µg/mL or more.
[211] The composition according to 209 or 210, which is in a form of a modified milk powder and comprises 0.1 mg or more of NMN per 100 g.

The present invention also provides the following items.
[301] A composition for reducing blood triglyceride levels in children, comprising a precursor of nicotinamide adenine dinucleotide (NAD).
[302] The composition according to 301, wherein the reduction in blood triglyceride levels in children is a reduction in blood triglyceride levels in infants or toddlers.
[303] The composition according to 301 or 302, wherein the reduction in blood triglyceride levels in children is a reduction in blood triglyceride levels in children after weaning.
[304] The composition according to any one of 301 to 303, which is to be taken by an infant or toddler.
[305] The composition according to any one of 301 to 303, which is to be taken by a child before weaning.
[306] The composition according to any one of 301 to 305, wherein the composition is in any form selected from the group consisting of a modified milk powder, modified liquid milk, and a breast milk substitute food.
[307] The composition according to any one of 301 to 306, wherein the precursor of NAD is nicotinamide mononucleotide (NMN).
[308] The composition according to 307, wherein the concentration of NMN during intake is 0.05 µg/ml or more.
[309] The composition according to 307 or 308, which is in a form of a modified milk powder and comprises 0.1 mg or more of NMN per 100 g.

The present invention also provides the following items.
[401] A composition for reducing blood glucose levels in children, comprising a precursor of nicotinamide adenine dinucleotide (NAD).
[402] The composition according to 401, wherein the reduction in blood glucose levels in children is a reduction in blood glucose levels in infants or toddlers.
[403] The composition according to 401 or 402, wherein the reduction in blood glucose levels in children is a reduction in blood glucose levels in children after weaning.
[404] The composition according to any one of 401 to 403, which is to be taken by an infant or toddler.
[405] The composition according to any one of 401 to 403, which is to be taken by a child before weaning.
[406] The composition according to any one of 401 to 405, wherein the composition is in any form selected from the group consisting of a modified milk powder, modified liquid milk, and a breast milk substitute food.
[407] The composition according to any one of 401 to 406, wherein the precursor of NAD is nicotinamide mononucleotide (NMN).
[408] The composition according to 407, wherein the concentration of NMN during intake is 0.05 µg/ml or more.
[409] The composition according to 407 or 408, which is in a form of a modified milk powder and comprises 0.1 mg or more of NMN per 100 g.

The present invention also provides the following items.
[501] A composition for inhibiting fat accumulation in children, comprising a precursor of nicotinamide adenine dinucleotide (NAD).
[502] The composition according to 501, wherein the inhibition of fat accumulation in children is inhibition of fat accumulation in infants or toddlers.
[503] The composition according to 501 or 502, wherein the inhibition of fat accumulation in children is inhibition of fat accumulation in children after weaning.
[504] The composition according to any one of 501 to 503, which is to be taken by an infant or toddler.
[505] The composition according to any one of 501 to 503, which is to be taken by a child before weaning.
[506] The composition according to any one of 501 to 505, wherein the composition is in any form selected from the group consisting of a modified milk powder, modified liquid milk, and a breast milk substitute food.
[507] The composition according to any one of 501 to 506, wherein the precursor of NAD is nicotinamide mononucleotide (NMN).
[508] The composition according to 507, wherein the concentration of NMN during intake is 0.05 µg/ml or more.
[509] The composition according to 507 or 508, which is in a form of a modified milk powder and comprises 0.1 mg or more of NMN per 100 g.

### Effects of the Invention

The composition of the present invention can contribute to the future healthy growth of children by improving exercise capacity of children, reducing blood triglyceride levels in children, reducing blood glucose levels in children, and inhibiting fat accumulation in children.

Since NMN, which is one of the active ingredients, is originally contained in breast milk, the intended effect can be safely exhibited in children.

The present invention provides information on the association of NMN taken before weaning with exercise capacity, blood triglyceride levels, blood glucose levels, and fat accumulation.

The composition of one embodiment can be expected to be applied to treatment of a disease or condition associated with exercise capacity of children. The composition of one embodiment can be expected to be applied to treatment of a disease or condition associated with blood triglyceride levels in children. The composition of one embodiment can be expected to be applied to treatment of a disease or condition associated with blood glucose levels in children. The composition of one embodiment can be expected to be applied to treatment of a disease or condition associated with fat accumulation in children.

In addition, the composition of the present invention can be expected to be applied to improvement in the nutritional status of children and maintenance/promotion of health.

### BRIEF DESCRIPTION OF THE DRAWINGS

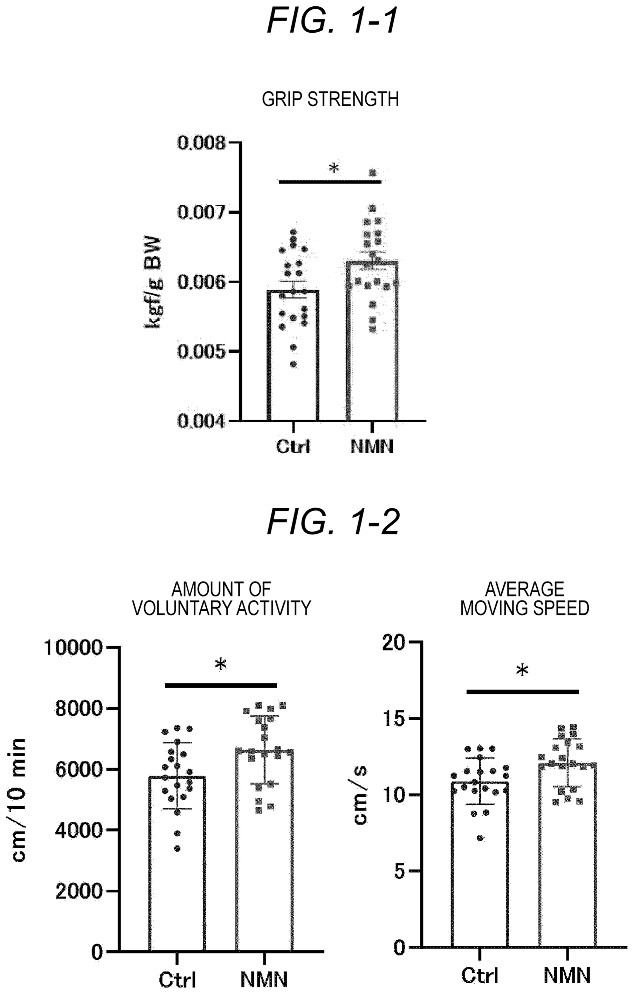

Fig. 1-1 shows grip strength. Three weeks after weaning, grip strength was measured. Ctrl: control group, NMN: NMN group, n = 20 in each group, Value: mean ± standard error, *: p < 0.05 Student's t-test vs Ctrl.
Fig. 1-2 shows the amount of voluntary activity and the average moving speed. Two weeks after weaning, an open field test was performed to measure the amount of voluntary activity and the average moving speed. Ctrl: control group, NMN: NMN group, n = 20 in each group, Value: mean ± standard error, *: p < 0.05 Student's t-test vs Ctrl.
Fig. 2 shows blood triglyceride concentration. Two weeks after weaning, plasma was isolated from blood obtained by whole blood collection, and the triglyceride concentration was measured. Ctrl: control group (n = 7), NMN: NMN group (n = 5), Value: mean ± standard error.
Fig. 3 shows blood glucose levels. Two weeks after weaning, plasma was separated from blood obtained by whole blood collection, and the blood glucose level was measured. Ctrl: control group (n = 7), NMN: NMN group (n = 5), Value: mean ± standard error, **:p < 0.01 Student's t-test vs Ctrl.
Fig. 4 shows relative weight of white fat. Two weeks after weaning, the mice were dissected, and the weight of white fat was measured. Ctrl: control group, NMN: NMN group, n = 10 in each group, Value: mean ± standard error, **:p < 0.01 Student's t-test vs Ctrl.

### MODES FOR CARRYING OUT THE INVENTION

### [Active ingredient]

The composition of the present embodiment comprises a precursor of nicotinamide adenine dinucleotide (NAD) as an active ingredient. The active ingredient refers to an ingredient that contributes to an object, and a food with functional claims is sometimes referred to as a functionally related component.

In the present invention, a precursor of NAD refers to NAD itself or a substance that can be converted to NAD in vivo. NAD functions as a coenzyme of various dehydrogenases in vivo, and can take two states of an oxidized form (NAD+) and a reduced form (NADH). In the present invention, NAD may be oxidized NAD+ or reduced NADH unless otherwise specified.

Only one kind of the precursor of NAD may be used in the composition, or two or more kinds thereof may be used.

In one embodiment, the composition comprises, as a precursor of NAD, any nicotinamide mononucleotide derivative that is a compound represented by the following general formula (I).

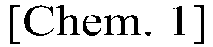

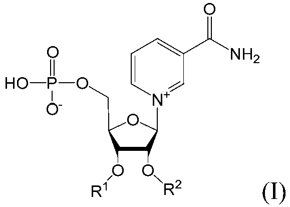

In the formula, R¹ and R² are each independently an acyl group having 6 to 16 carbon atoms, and a hydrocarbon group bonded to a carbonyl carbon of the acyl group is a linear or branched saturated or unsaturated hydrocarbon group. Such a compound can be produced by acylating NMN in a solvent comprising 20 mass% or more of a strongly acidic liquid with a pKa of 2.0 or less using any acylating agent selected from the group consisting of carboxylic acids having an acyl group having 6 to 16 carbon atoms in which a linear or branched saturated or unsaturated hydrocarbon group is bonded to a carbonyl carbon, halides of the carboxylic acids, and anhydrides of the carboxylic acids (see WO 2017/110317 A).

In one embodiment, the composition includes, as a precursor of NAD, any one selected from the group consisting of nicotinamide mononucleotide (NMN), nicotinic acid, nicotinamide (may also be referred to as niacinamide), nicotinamide riboside, reduced nicotinamide riboside, β-trigonelline, nicotinic acid mononucleotide, nicotinic acid riboside, tryptophan, and salts thereof acceptable as a food or a medicine.

In a preferred embodiment, the composition comprises, as a precursor of NAD, NMN or a salt thereof acceptable as a food or a medicine.

There are two types of NMN, i.e., an α-form and a β-form, as optical isomers. In the present invention, NMN refers to β-form NMN (β-nicotinamide mononucleotide) unless otherwise specified. NMN is an intermediate metabolite of NAD+.

The salt acceptable as a food or a medicine refers to any salt selected from the group consisting of nitrates, sulfates, carbonates, hydrogen carbonates, halogen salts, formates, acetates, citrates, tartrates, oxalates, fumarates, salts of saturated or unsaturated fatty acids having 3 to 20 carbon atoms, salts of carnitine and derivatives thereof, salts of hydroxycitric acid and derivatives thereof, salts of ascorbic acid and derivatives thereof, salts of ascorbyl phosphoric acid and derivatives thereof, sodium salts, potassium salts, calcium salts, magnesium salts, zinc salts, and ammonium salts.

The precursor of NAD, NMN, nicotinic acid, nicotinamide, nicotinamide riboside, reduced nicotinamide riboside, β-trigonelline, nicotinic acid mononucleotide, nicotinic acid riboside, tryptophan, and the salt acceptable as a food or a medicine thereof can be produced by various methods. It may be synthesized, or it may be extracted from a natural product or a culture such as yeast comprising the substance.

### [Application]

The composition of the present embodiment is used for any one selected from improvement in exercise capacity of children, reduction in blood triglyceride levels in children, reduction in blood glucose levels in children, and inhibition of fat accumulation in children. In the present invention, the term "any" means that number and type are arbitrary.

In the present invention, the term "child (children)" is not limited to a human child (children) unless otherwise specified. In the present invention, the term "age" refers to the age of a human unless otherwise specified or determined to be the age of a specific animal from context or the like. For example, when simply referred to as 3 years old, it refers to 3 years old in humans, and when the subject is a mouse, it is about 4 weeks old, which corresponds to 3 years old in humans. Age relationship for humans and mice can be referenced by S. Dutta, P. Sengupta. Life Sciences 152 (2016) 244-248. In the present invention, the term "child (children)" refers to a person aged less than 18 years old in humans, including those who are before weaning, during weaning, and for a while after weaning (for example, from after weaning to about 3 years old), and includes toddlers.

Weaning refers to the period during which a nutrient source other than breast milk or a substitute thereof is taken together with breast milk or a substitute thereof. In the case of a human, weaning is often between the 4 to 6 months old and about one and a half years old with individual differences. The term "before weaning" refers to before weaning and during weaning, and the term "after weaning" does not include during weaning but refers to after weaning.

In the present invention, treatment for a disease or condition includes reduction in a risk of onset, delay of onset, prevention, treatment, and stop and delay of progression. The treatments include medical actions performed by a doctor for the purpose of treating a disease, and non-medical actions performed by a person other than a doctor, for example, a dietitian, a national registered dietitian, a public health nurse, a midwife, a nurse, a clinical laboratory technician, a food manufacturer, a food seller, or the like. The treatments also include recommendation of administration or intake of specific foods, dietary guidance, health guidance, nutritional guidance (including instruction of nutrition necessary for medical care for a sick/injured person and instruction of nutrition for promoting health maintenance), meal management, guidance related to meal necessary for nutrition improvement. The subjects to be treated in the present invention include humans (individuals) and non-human mammals (companion animals and the like). Also in the present invention, the term "intake (take)" can be read as the term "administration (administer)". The term "administration (administer)" may be used not only for pharmaceutical compositions but also for food compositions.

### (Function/action)

### <Improvement in exercise capacity>

In one embodiment, the composition is used for improving exercise capacity of children.

According to the study of the present inventors, it has been found that a mouse administered with NMN in infancy, more specifically, a mouse administered with NMN until day 21 after birth (date of weaning) has increased grip strength after 3 weeks from weaning (according to the above document, it is calculated to be 4.6 years old in humans.) as compared to a control mouse.

Therefore, in one embodiment, the composition is used to be taken by a child at any time selected from the group consisting of before weaning, during weaning, and for a while after weaning (for example, from after weaning to about 3 years old), preferably before weaning (before weaning and during weaning).

In another embodiment, the composition is used for improving exercise capacity of children after the intake period. For example, the composition is used for improving exercise capacity of children at any time selected from after weaning, for a while after weaning (e.g., from after weaning to about 3 years old), or 3 to 5 years old. Preferably, the composition is used for improving the exercise capacity of children after weaning.

In a particularly preferred embodiment, the composition is taken by a child at a certain time and used for improving the exercise capacity of the child after that time. For example, the composition is taken by a child at any time selected from before weaning, during weaning, and for a while after weaning (for example, from after weaning to about 3 years old), and is used for improving the exercise capacity of the child at any time selected from after weaning, for a while after weaning (e.g., from after weaning to about 3 years old), and 3 to 5 years old. Preferably, the composition is taken by a child before weaning and used for improving the exercise capacity of the child after weaning.

The improvement in exercise capacity includes promotion of skeletal muscle development and improvement in activity. The promotion of skeletal muscle development can be evaluated by improvement in muscle strength. The activity refers to being active. The improvement in activity can be evaluated by, for example, an increase in the amount of voluntary activity and an increase in the average moving speed. The improvement in activity includes the case of improvement in brain function (central nervous system such as nerves).

The improvement in exercise capacity can be evaluated by, for example, pulling to stand, cruising, crouching, walking alone, walking with alternately swinging arms and legs, standing on one leg, stair climbing, jumping with both feet, hanging with both hands, running, throwing, kicking, and the like.

The improvement in exercise capacity can also be evaluated by, for example, 25 m running, standing long jump, ball throwing, jumping continuously with both feet, body support duration, and catching. The improvement in exercise capacity can also be evaluated by, for example, grip strength, upper body raising, long seat body anteflexion, repeated lateral jumping, 20 m shuttle run, 50 m running, standing long jump, and softball throwing.

In one embodiment, the composition of the present invention is used for maintaining exercise capacity, for excellently maintaining the improvement in exercise capacity, and for aiding them.

In one embodiment, the composition of the present invention is used for treatment of a disease or condition associated with exercise capacity, which can be expected to be better by improving exercise capacity.

### <Reduction in blood triglyceride levels>

In one embodiment, the composition is used for reducing blood triglyceride levels in children. Blood triglycerides may also be referred to as neutral fats.

Triglycerides are lipids in foods such as meat, fish, and edible oils, and substances that occupy most of body fat, and are also simply referred to as fats. Triglycerides are an important energy source and are also essential for the intake of fat-soluble vitamins and essential fatty acids, but if taken too much, they are stored as body fat, leading to obesity and causing lifestyle diseases. Generally, when the triglyceride concentration in the blood is 150 mg/dL or more, it is considered as hypertriglyceridemia.

According to the study of the present inventors, it has been found that a mouse administered with NMN in infancy, more specifically, a mouse administered with NMN until day 21 after birth (date of weaning) tends to have a lower blood triglyceride concentration after 2 weeks from weaning (according to the above document, it is calculated to be 3.8 years old in humans.) as compared to a control mouse.

Therefore, in one embodiment, the composition is used to be taken by a child at any time selected from the group consisting of before weaning, during weaning, and for a while after weaning (for example, from after weaning to about 3 years old), preferably before weaning (before weaning and during weaning).

In another embodiment, the composition is used for reducing blood triglyceride levels in children after the intake period. For example, the composition is used for reducing blood triglyceride levels in children at any time selected from after weaning, for a while after weaning (e.g., from after weaning to about 3 years old), or 3 to 5 years old. Preferably, the composition is used for reducing blood triglyceride levels in children after weaning.

In a particularly preferred embodiment, the composition is taken by a child at a certain time and used for reducing blood triglyceride levels in the child after that time. For example, the composition is taken by a child at any time selected from before weaning, during weaning, and for a while after weaning (for example, from after weaning to about 3 years old), and is used for reducing blood triglyceride levels in the child at any time selected from after weaning, for a while after weaning (e.g., from after weaning to about 3 years old), and 3 to 5 years old. Preferably, the composition is taken by a child before weaning and used for reducing blood triglyceride levels in the child after weaning.

The reduction in blood triglyceride levels can be measured using, for example, a commercially available kit using separated plasma.

In one embodiment, the composition of the present invention is used for maintaining blood triglyceride levels, for excellently maintaining reduced blood triglyceride levels, and for aiding them.

In one embodiment, the composition of the present invention is used for treatment of a disease or condition associated with blood triglyceride levels, which can be expected to be better by reducing blood triglyceride levels.

### <Reduction in blood glucose level>

In one embodiment, the composition is used for reducing blood glucose levels in children.

The blood glucose level is the concentration of glucose in the blood. Carbohydrates and the like in the diet are digested and absorbed as glucose into the blood. Human blood glucose levels are kept in a relatively narrow range (about 70 to 100 mg/dL in a fasting state) by the action of insulin, glucagon, and the like. A state in which the blood glucose levels stay high and do not decrease is called hyperglycemia, and if this state continues for a long time, there is a risk that the blood vessel is injured and arteriosclerosis occurs. Diabetes is a disease in which the blood glucose levels are chronically high since insulin secretion is insufficient or insulin does not function adequately even if insulin is secreted. In recent years, since the number of children with type 2 diabetes is increasing, it can be expected that the fact that the blood glucose levels can be reduced and kept low has a preventive role on the onset of diabetes.

According to the study of the present inventors, it has been found that a mouse administered with NMN in infancy, more specifically, a mouse administered with NMN until day 21 after birth (date of weaning) has a significantly lower blood glucose level after 2 weeks from weaning (according to the above document, it is calculated to be 3.8 years old in humans.) as compared to a control mouse.

Therefore, in one embodiment, the composition is used to be taken by a child at any time selected from the group consisting of before weaning, during weaning, and for a while after weaning (for example, from after weaning to about 3 years old), preferably before weaning (before weaning and during weaning).

In another embodiment, the composition is used for reducing blood glucose levels in children after the intake period. For example, the composition is used for reducing blood glucose levels in children at any time selected from after weaning, for a while after weaning (e.g., from after weaning to about 3 years old), or 3 to 5 years old. Preferably, the composition is used for reducing blood glucose levels in children after weaning.

In a particularly preferred embodiment, the composition is taken by a child at a certain time and used for reducing blood glucose levels in the child after that time. For example, the composition is taken by a child at any time selected from before weaning, during weaning, and for a while after weaning (for example, from after weaning to about 3 years old), and is used for reducing blood glucose levels in the child at any time selected from after weaning, for a while after weaning (e.g., from after weaning to about 3 years old), and 3 to 5 years old. Preferably, the composition is taken by a child before weaning and used for reducing blood glucose levels in the child after weaning.

The reduction in blood glucose levels can be measured using, for example, a commercially available kit using separated plasma.

In one embodiment, the composition of the present invention is used for maintaining blood glucose levels, for excellently maintaining the reduction in blood glucose levels, and for aiding them.

In one embodiment, the composition of the present invention is used for treatment of a disease or condition associated with blood glucose levels, which can be expected to be better by reducing blood glucose levels.

### <Inhibition of fat accumulation>

In one embodiment, the composition is used for inhibiting fat accumulation in children.

Generally, regarding the body, when referring to fat, visceral fat and subcutaneous fat are included. According to the study of the present inventors, since the white fat around the reproductive organs after weaning of the mouse administered with NMN in infancy was lower than that of the control mouse, the composition of the present invention is suitable for inhibiting accumulation of particularly visceral fat among fats.

According to the study of the present inventors, it has been found that a mouse administered with NMN in infancy, more specifically, a mouse administered with NMN until day 21 after birth (date of weaning) tends to have a lower fat accumulation concentration after 2 weeks from weaning (according to the above document, it is calculated to be 3.8 years old in humans.) as compared to a control mouse.

Therefore, in one embodiment, the composition is used to be taken by a child at any time selected from the group consisting of before weaning, during weaning, and for a while after weaning (for example, from after weaning to about 3 years old), preferably before weaning (before weaning and during weaning).

In another embodiment, the composition is used for inhibiting fat accumulation in children after the intake period. For example, the composition is used for inhibiting fat accumulation in children at any time selected from after weaning, for a while after weaning (e.g., from after weaning to about 3 years old), or 3 to 5 years old. Preferably, the composition is used for inhibiting fat accumulation in children after weaning.

In a particularly preferred embodiment, the composition is taken by a child at a certain time and used for inhibiting fat accumulation in the child after that time. For example, the composition is taken by a child at any time selected from before weaning, during weaning, and for a while after weaning (for example, from after weaning to about 3 years old), and is used for inhibiting fat accumulation in the child at any time selected from after weaning, for a while after weaning (e.g., from after weaning to about 3 years old), and 3 to 5 years old. Preferably, the composition is taken by a child before weaning and used for inhibiting fat accumulation in the child after weaning.

The amount or degree of accumulation of fat in the body can be measured by, for example, a CT device or a body fat meter (electrical conductivity method/bioimpedance method). In addition, it can be estimated by measuring an abdominal circumference that is said to be substantially proportional to accumulation of visceral fat.

In one embodiment, the composition of the present invention is used for not accumulating fat, for excellently maintaining a state in which fat accumulation is inhibited, and for aiding them.

In one embodiment, the composition of the present invention is used for treatment of a disease or condition associated with fat accumulation, which can be expected to be better by inhibiting fat accumulation.

### (Subject)

The composition of the present embodiment is used to be taken by a child (less than 18 years old). In the present invention, the term "child (children)" refers to an infant (less than 1 year old, including neonates up to 1 month old), a toddler (1 year old or more and less than 6 years old), and a juvenile (6 years old or more and less than 18 years old) unless otherwise specified.

The composition of one embodiment is suitable to be taken by a subject in which an improvement in exercise capacity is desired or in need thereof. The composition of one embodiment can also be expected to be applied to treatment of a disease or condition associated with exercise capacity of children. The composition of one embodiment is suitable to be taken by a subject in which a reduction in blood triglyceride levels is desired or in need thereof. The composition of one embodiment can also be expected to be applied to treatment of a disease or condition associated with blood triglyceride levels in children. The composition of one embodiment is suitable to be taken by a subject in which a reduction in blood glucose levels is desired or in need thereof. The composition of one embodiment can also be expected to be applied to treatment of a disease or condition associated with blood glucose levels in children. The composition of one embodiment is suitable to be taken by a subject in which an inhibition of fat accumulation is desired or in need thereof. The composition of one embodiment can also be expected to be applied to treatment of a disease or condition associated with fat accumulation in children.

In one embodiment, the composition is used to be taken by an infant before weaning or a toddler. In yet another embodiment, the composition of the present invention is used for any one selected from improvement in exercise capacity, reduction in blood triglycerides, reduction in blood glucose levels, and inhibition of fat accumulation, in infants or toddlers. In a particularly preferred embodiment, the composition of the present invention is taken by an infant before weaning or a toddler, and is used for any one selected from subsequent improvement in exercise capacity, reduction in blood triglycerides, reduction in blood glucose levels, and inhibition of fat accumulation in the child.

In one embodiment, the composition is suitable to be taken by a subject in which formula feeding rather than breast feeding is desired or a subject in need of formula feeding. The composition is also suitable to be taken by non-human animals.

### [Composition]

### (Dosage form/form)

The composition of the present embodiment can be prepared in any form such as a solid, a liquid, a mixture, a suspension, an emulsion, a gel, or a sol. The composition of the present embodiment can be a synthesized nutritional composition prepared by mixing various components. The synthesized nutritional composition may be simply referred to as a nutritional composition. The nutritional composition does not comprise biosynthetic composition such as breast milk. In the present invention, the composition (including nutritional composition) for children refers to a composition to be taken by children, and does not include a composition to be taken by pregnant women and a composition to be taken by lactating women.

In a particularly preferred embodiment, the composition is in a form of a modified milk powder, modified liquid milk, or a breast milk substitute food. The modified milk powder refers to raw milk, cow's milk, or special cow's milk, or a food produced using these as raw materials, processed or used as a main raw material, to which nutrients necessary for infants and toddlers are added to form a powder. The modified liquid milk refers to raw milk, cow's milk, or special cow's milk, or a food produced using these as raw materials, processed or used as a main raw material, to which nutrients necessary for infants and toddlers are added to form a liquid.

The modified milk powder, modified liquid milk, or breast milk substitute food may be those for infants, for follow-up, for low birth weight infants, for children, for adults, for the elderly, for allergies, for lactose intolerance, or for inborn errors of metabolism. In Japan, the term "for follow-up" refers to those for taking in nutrients required from infancy to toddlerhood in a well-balanced manner from weaning from about 9 months old to about 3 years old.

In one embodiment, the modified milk powder is used by being dissolved so as to be 5 to 17%, preferably 8 to 16%, and more preferably 12 to 15%. The modified milk powder can be in a form of a stick package or cuboid in a single volume (for example, a volume for dissolving in hot water to prepare 200 ml of milk).

The composition of the present embodiment can be a composition that is a food or a medicine other than a modified milk powder, modified liquid milk, or a breast milk substitute food. The food or the medicine includes not only one for humans but also one for animals other than humans, unless otherwise stated. The food includes, unless otherwise stated, a general food, a functional food, an infant food, and a nutritional composition, and includes a therapeutic meal (a meal serving therapeutic purposes, or a meal cooked based on a menu prepared by a dietitian or the like in accordance with a dietary recipe presented by a doctor), a dietary meal, an ingredient adjustment meal, a nursing meal, and a food for treatment support. The food includes not only solid products but also liquid products such as beverage, health drink, liquid food, and soup, unless otherwise specified. The functional food refers to foods that can impart a predetermined functionality to a living body, and generally include health foods, for example, food for specified health uses (including conditional food for specified health uses [Tokuho]), food with function claims, food with health claims including food with nutrient function claims, food for special dietary uses, nutritional supplement food, health supplement food, supplement (for example, those of various kinds of dosage forms such as tablet, coated tablet, sugar-coated tablet, capsule, and liquid drug), cosmetic food (for example, diet food), and so forth. In addition, in the present invention, the functional food includes health foods to which a health claim based on the food standard of Codex (Joint FAO/WHO Codex Alimentarius Commission, CAC) is applied. Cosmetics include medicinal cosmetics.

When the composition of the present embodiment is a food, it can be in any form such as a supplement, a confection, a beverage, a health drink, a seasoning, a milk product, a processed food, a daily dish, or a soup. Specifically, the composition of the present invention can be in a form of a gummy, a tablet, a health drink, fermented milk, a dairy beverage, a milk beverage, a soft drink, ice cream, a cheese, bread, a biscuit, a cracker, a pizza crust, modified milk powder, a liquid food, a food for sick persons, a nutritional food, a frozen food, a processed food, or the like, can be in a form of a granule, a powder, a paste, a concentrated liquid, or the like for mixing with a beverage or a food, or for preparing a beverage or the like by dilution, and specifically can be in a form of syrup for adding to milk.

When the composition of the present embodiment is a medicine, specific form examples thereof include chewable tablets, sublingual tablets, tablets, pills, capsules, granules, powders, powdered medicines, solutions, suspensions, emulsions, syrups, liniments, lotions, gels, aerosols, sprays, ointments, creams, tapes, cataplasms, eye drops, nasal drops, and suppositories.

### (Content and dose of active ingredient)

The content and concentration of the precursor of NAD of the composition of the present embodiment (when a plurality of precursors of NAD are used, the total content and concentration of the precursors of NAD) may be any amount as long as the intended effect is exhibited, and can be appropriately set in consideration of various factors such as the age in month, age, and body weight of the subject, and the intended effect. In the present invention, when the amount or concentration of the precursor of NAD in the composition is indicated, the amount or concentration as NMN is indicated unless otherwise specified. When a precursor of NAD other than NMN is used as an active ingredient, a precursor of NAD in an equimolar amount/equimolar concentration with the amount or concentration of NMN may be used. This conversion can be easily performed by those skilled in the art. In the following description, the amount or concentration will be described by exemplifying a case where NMN is used as a precursor of NAD, but those skilled in the art can understand the description by appropriately applying to a case where a precursor of NAD other than NMN is used.

When the composition of the present embodiment is a solid, for example, a modified milk powder, the amount of NMN per 100 g of the composition can be 0.05 mg or more, and is preferably 0.1 mg or more, more preferably 0.5 mg or more, and still more preferably 1 mg or more. The amount of NMN per 100 g of the composition may also be 1.1 mg or more, 1.5 mg or more, 2 mg or more, 2.5 mg or more, 3 mg or more, 4 mg or more, 4.5 mg or more, 5 mg or more, 6 mg or more, 7 mg or more, 8 mg or more, 9 mg or more, or 10 mg or more.

When the composition of the present embodiment is a solid, for example, a modified milk powder, the upper limit of the amount of NMN per 100 g of the composition can be 200 mg or less, 100 mg or less, 50 mg or less, or 10 mg or less. From the viewpoint of being within the range of the amount usually comprised in breast milk, the amount of NMN per 100 g of the composition may be 16.1 mg or less, or 13 mg or less.

When the composition of the present embodiment is a liquid, for example, modified liquid milk, the NMN concentration of the composition can be 0.025 µg/mL or more, and is preferably 0.05 µg/mL or more, more preferably 0.25 µg/mL or more, still more preferably 0.5 µg/mL or more, still more preferably 0.85 µg/mL or more, and still more preferably 1.7 µg/mL or more. The NMN concentration may also be 1.8 µg/mL or more, 2 µg/mL or more, 2.2 µg/mL or more, 2.4 µg/mL or more, 2.6 µg/mL or more, 3 µg/mL or more, 3.5 µg/mL or more, 3.7 µg/mL or more, 4 µg/mL or more, 4.3 µg/mL or more, 4.6 µg/mL or more, 5 µg/mL or more, or 5.5 µg/mL or more.

When the composition of the present embodiment is a liquid, for example, modified liquid milk, the upper limit of the NMN concentration of the composition can be 340 µg/mL or less, 170 µg/mL or less, 85 µg/mL or less, 25 µg/mL or less, 17 µg/mL or less, or 5 µg/mL or less. From the viewpoint of being within the range of the amount usually contained in breast milk, the concentration of NMN is preferably 8.1 µg/mL or less, and may be 6.0 µg/mL or less or 3.1 µg/mL or less.

The content of NMN in the composition of the present embodiment may be designed in consideration of the amount of NMN per day. The daily dose of NMN can be 0.013 mg or more, and is preferably 0.25 mg or more, more preferably 0.43 mg or more, and still more preferably 0.85 mg or more. The daily dose of NMN also may be 0.9 mg or more, 1 mg or more, 1.2 mg or more, 1.6 mg or more, 2 mg or more, 3 mg or more, 6 mg or more, 6.3 mg or more, 6.6 mg or more, 7 mg or more, 7.5 mg or more, or 8 mg or more.

The upper limit of the daily dose of NMN can be 170 mg or less, and may be 85 mg or less, 43 mg or less, 13 mg or less, 8.5 mg or less, or 2.5 mg or less. From the viewpoint of being within the range of the amount usually contained in breast milk, the daily dose of NMN is preferably 8.1 mg or less, and may be 6.0 mg or less.

Such a daily dose may be divided into a plurality of portions, for example, 3 to 7 portions so as to be suitable for intake 3 to 7 times a day.

The composition of the present embodiment may be taken at any time of the day. When the composition of the present embodiment is a modified milk powder, modified liquid milk, or a breast milk substitute food, it can be fed using the composition of the present embodiment as in the case of breastfeeding.

It is considered that the NAD concentration in the body has a daily variation, and from the viewpoint of increasing in the active period, in one embodiment, it is considered preferable that the composition is taken more in the morning at the start of the active period.

### (Other components, additives)

In addition to the precursor of NAD, the composition of the present embodiment may comprise other nutritional components acceptable as a food or a medicine. For example, when the composition is in a form of a modified milk powder, modified liquid milk, or a breast milk substitute food, the composition may comprise, as components other than NMN, protein, lipid, cholesterol, carbohydrate, niacin, pantothenic acid, biotin, vitamin A, vitamin B1, vitamin B2, vitamin B6, vitamin B12, vitamin C, vitamin D, vitamin E, vitamin K, folic acid, zinc, sodium, potassium, calcium, selenium, iron, copper, magnesium, phosphorus, lactadherin, α-lactalbumin, β-lactoglobulin, ARA (arachidonic acid), DHA (docosahexaenoic acid), linoleic acid, α-linolenic acid, phospholipids, fructo-oligosaccharides, inositol, β-carotene, chlorine, carnitine, taurine, nucleotides, and the like.

In addition to the precursor of NAD, the composition of the present invention may also comprise other active ingredients and nutritional components acceptable as a food or a medicine.

In addition, the composition of the present embodiment may further comprise an additive acceptable as a food or a medicine. Examples of such additive are an inert carrier (solid or liquid carrier), an excipient, a surfactant, a binder, a disintegrant, a lubricant, a solubilizing agent, a suspending agent, a coating, a colorant, a preservative, a buffering agent, a pH adjusting agent, an emulsifier, a stabilizer, a sweetener, an antioxidant, a flavor, an acidulant, and a natural product.

### (Usage)

The composition of the present embodiment is used orally. The composition may be administered parenterally, for example, via a tube (e.g., gastrostomy, jejunostomy) or used nasally, but it is desirable to use it orally.

The composition of the present embodiment can be repeatedly taken by a subject, and can be continuously taken by a subject for a long period. The period is not particularly limited, and in order to sufficiently exhibit the effect, it is preferable that the composition is continuously taken for a relatively long period, for example, 1 week or more, 2 weeks or more, 1 month or more, 3 months or more, 6 months or more, or 1 year or more. Since NMN as an active ingredient is a substance having a long food experience and NMN is originally contained in breast milk, the composition of the present embodiment is particularly suitable to be taken for a long period.

### (Others)

The composition of the present embodiment is used together with intake of other components, intake of health foods, supplements, and the like, exercise, normal diet, and the like. In addition, the composition of the present embodiment may be used in combination with any one selected from a therapy for promoting improvement in exercise capacity, a therapy for promoting reduction in blood triglycerides, a therapy for promoting reduction in blood glucose level, and a therapy for promoting inhibition of fat accumulation.

The composition of the present embodiment can be appropriately produced by those skilled in the art using existing equipment and the like. In the production of the composition of the present embodiment, the stage of NMN blending is not particularly limited as long as the properties of NMN are not significantly impaired.

The composition of the present embodiment can be produced according to a conventional method even when it is in a form of a modified milk powder, modified liquid milk, or a breast milk substitute food. For example, in the case of a modified milk powder, it can be produced as follows. A modified milk powder can be obtained by blending a processed product of whey protein, casein, a processed product of soybean protein, saccharides (lactose, oligosaccharide), minerals, vitamins, and oils and fats (including ARA, DHA, linoleic acid, α-linolenic acid or the like as a fatty acid), dissolving, mixing, and cleaning the mixture, then sterilizing, concentrating, and homogenizing the mixture, and subjecting the mixture to spray drying and other drying steps. Also, in this case, the stage of NMN blending is not particularly limited as long as the properties of NMN are not significantly impaired.

In a product compriseing the composition of the present embodiment, the function or intended use (application) can be indicated, and a recommendation of administration and intake to a specific subject can be indicated. The indication can be a direct or indirect indication. Examples of the direct indication include indications on tangible objects such as the product itself, package, container, label, tag, of the product, etc. Examples of the indirect indication include advertisement or publicity on such places or by such means as website, storefront, pamphlet, exhibition, seminar such as media seminar, book, newspaper, magazine, television, radio, mail, e-mail, and voice. Examples of the function or intended use (application) to be indicated include "helping to improve exercise capacity", "helping to maintain exercise capacity", "helping to reduce blood triglyceride levels", "helping to maintain blood triglyceride levels low", "helping to reduce blood glucose levels", "helping to maintain blood glucose levels low", "helping to inhibit fat accumulation", "for babies up to 6 months old", "for infants", "for toddlers", and the like.

### EXAMPLES

It was verified whether or not the NMN administration to infantile mice affects the development of exercise capacity, blood triglyceride levels, blood glucose levels, and white fat accumulation (lipid metabolism) after weaning.

### [Examples 1 to 4: Common method]

### 1) Substance to be administered

β-Nicotinamide mononucleotide (NMN) to be administered to mice was dissolved at a concentration of 100 mg/mL in distilled water and then used for administration.

### 2) Animal

Ten female C57BL/6J lines at day 13 of gestation were purchased. The mice were individually raised in a breeding room at a room temperature of 22 ± 2°C, humidity of 55 ± 15%, light period of 7:00 to 19:00, and dark period of 19:00 to 7:00 until the birth of the pups, and after the birth, group breeding was performed with the pups until weaning, and the born mouse pups were used for the test.

### 3) Administration of NMN

All the mouse pups were subjected to sex determination on day 3 after birth, and were divided into distilled water administration (later control group) and NMN administration (later NMN group) so that the number of males and females was approximately half in the littermates. The individuals of NMN administration were orally gavaged with an aqueous NMN solution at a dosage volume of 10 µL/body per day from days 3 to 13 after birth, and a dosage volume of 5 mL/kg BW (dosage amount: 500 mg/kg BW) from days 14 to 21 (date of weaning). The individuals of distilled water administration were orally gavaged with distilled water at the same dosage and frequency.

### [Example 1: Influence on development of exercise capacity after weaning]

### (Method)

### 1) Grouping

The body weights of all the mouse pups were measured on the day of weaning, and the pups were divided into a total of four groups: a control group and an NMN group for males and a control group and an NMN group also for females (n = 10 for each group), so that the average values of the body weights were equal. After weaning, normal breeding was performed until the measurement date of the grip strength, the amount of voluntary activity, and the average moving speed.

### 2) Measurement of grip strength

Three weeks after weaning, the grip strength (forelimb only) of all the individuals was measured using Grip Strength Meter for Rats & Mice (MK-380CM/F, manufactured by Muromachi Kikai Co., Ltd.). The measurement was performed 10 times per individual, and the maximum value was recorded. The grip strength was corrected by the body weight of each individual to calculate the value.

### 3) Measurement of amount of voluntary activity and average moving speed

Two weeks after weaning, an open field test was performed on all the individuals to measure the amount of voluntary activity and the average moving speed. The mice were placed in an open field bath (manufactured by SHIN FACTORY), and free movement for 10 minutes was video-taped. From the obtained moving image, the amount of voluntary activity (total movement distance) and the average moving speed (average of moving speed excluding immobility state) were calculated using dedicated analysis software (SMART3.0: manufactured by Panlab).

### (Results)

### 1) Grip strength

In the NMN group, the grip strength compared when males and females were combined was high as compared to that in the control group (Fig. 1-1).

### 2) Amount of voluntary activity and average moving speed

In the NMN group, the amount of voluntary activity and the average moving speed compared when males and females were combined were high as compared to those in the control group (Fig. 1-2).

### (Discussion)

By the administration of NMN in infancy, all of the grip strength, the amount of voluntary activity, and the average moving speed after weaning were increased. It has already been reported that in mice in which SIRT1 (Feldman JL, Dittenhafer-Reed KE, Denu JM. J Biol Chem. 2012; 287: 42419-42427), which functions as a coenzyme of NAD+, is highly expressed in the brain, skeletal muscle function is improved and the amount of voluntary activity is increased (Non Patent Document 1 described above), and exercise capacity is improved in clinical trials involving NMN (Non Patent Document 2 described above). Therefore, in this test, it was considered possible that by administering NMN in infancy, the amount of NAD+ in the brain and the skeletal muscle increased, and the growth and development of the skeletal muscle were promoted, so that the exercise capacity was improved through an increase in muscle strength and the like.

### (Conclusion)

It has been demonstrated that the administration of NMN in infancy improves exercise capacity such as grip strength, amount of voluntary activity, and average moving speed after weaning. The present invention leads to elucidation of physiological significance of NMN contained in breast milk, and is useful for healthy growth and development of many children.

### [Example 2: Influence on blood triglyceride levels after weaning]

### (Method)

### 1) Grouping

The body weights of the mouse pups were measured on the day of weaning, and the pups were divided into a total of two groups: a control group (n = 7) and an NMN group (n = 5) only for males. After weaning, the mice were normally bred for 2 weeks, and plasma was separated from blood obtained by whole blood collection.

### 2) Measurement of triglyceride concentration

Using the separated plasma, the triglyceride concentration was measured by a kit (Triglyceride E-test Wako: No. 432-40201).

### (Results)

### Blood triglyceride concentration

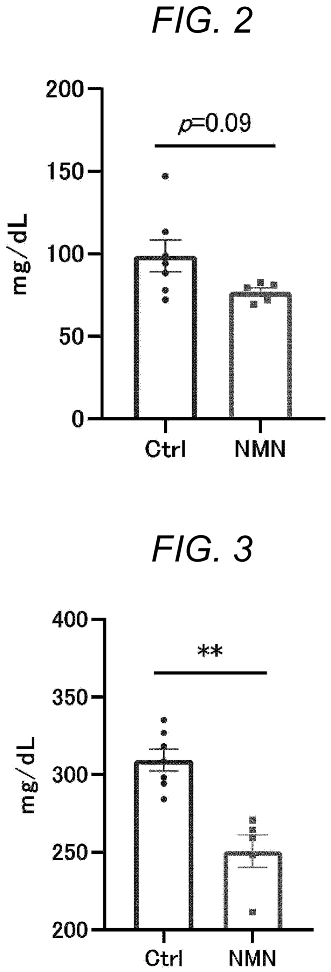

In the NMN group, the blood triglyceride concentration tended to be low as compared to that in the control group (Fig. 2).

### (Discussion)

By the administration of NMN in infancy, the blood triglyceride concentration after weaning was reduced. It has already been reported that in a test in which NMN is administered to mice after feeding a high-fat diet, lipid metabolism is improved, and the amount of triglycerides in the liver is reduced (Non Patent Document 3 described above). Therefore, in this test, it was considered that the blood triglyceride concentration was reduced by affecting the lipid metabolism by administering NMN in infancy.

### (Conclusion)

It has been demonstrated that the administration of NMN in infancy reduces blood triglycerides after weaning. The present invention leads to elucidation of physiological significance of NMN contained in breast milk, and is useful for healthy growth and development of many children.

### [Example 3: Influence on blood glucose levels after weaning]

### (Method)

### 1) Grouping

The body weights of the mouse pups were measured on the day of weaning, and the pups were divided into a total of two groups: a control group (n = 7) and an NMN group (n = 5) only for males. After weaning, the mice were normally bred for 2 weeks, and plasma was separated from blood obtained by whole blood collection.

### 2) Measurement of blood glucose level

Using the separated plasma, the blood glucose concentration (blood glucose level) was measured by a kit (Glucose II-test Wako: No. 439-90901).

### (Results)

### Blood glucose level

In the NMN group, the blood glucose levels were significantly low as compared to those in the control group (Fig. 3).

### (Discussion)

By the administration of NMN in infancy, the blood glucose levels after weaning were low. It has already been reported that in the test in which NMN is administered to mice after feeding a high-fat diet, glucose tolerance is improved, and the production amount of incretin (GLP-1) in gastrointestinal tissues is increased (Non Patent Documents 3 and 4). Therefore, in this test, it was considered that the blood glucose levels were reduced by affecting the glucose tolerance (glucose metabolism) by administering NMN in infancy.

### (Conclusion)

It has been demonstrated that the administration of NMN in infancy reduces blood glucose levels after weaning. The present invention leads to elucidation of physiological significance of NMN contained in breast milk, and is useful for healthy growth and development of many children.

### [Example 4: Influence on accumulation of white fat (lipid metabolism) after weaning]

### (Method)

### Grouping, white fat weight measurement

The body weights of all the mouse pups were measured on the day of weaning, and the pups were divided into a total of four groups: a control group (n = 7) and an NMN group (n = 5) for males, and a control group (n = 3) and an NMN group (n = 5) also for females. After weaning, the mice were normally bred for 2 weeks and dissected, the white adipose tissue around the reproductive organ was extracted, and the weight thereof was measured. The white fat weight was corrected by the body weight of each individual to calculate the relative weight value.

### (Results)

### White fat weight

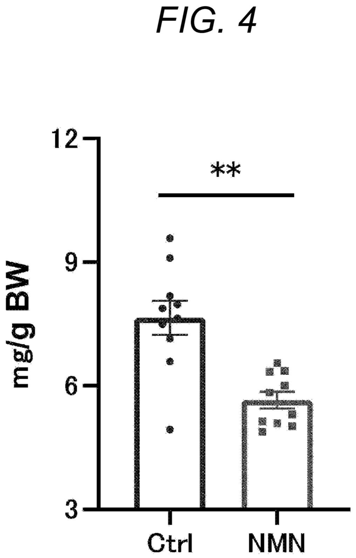

In the NMN group, the white fat weight when males and females were combined was low as compared to that in the control group (Fig. 4).

### (Discussion)

By the administration of NMN in infancy, the white fat weight after weaning was inhibited. It has already been reported that in the test in which NMN is administered to mice after feeding a high-fat diet, glucose tolerance and lipid metabolism are improved, and the production amount of incretin (GLP-1) in gastrointestinal tissues is increased (Non Patent Documents 3 and 4 described above). Therefore, in this test, it was considered that the accumulation of white fat was inhibited by affecting the glucose tolerance and the lipid metabolism by administering NMN in infancy.

### (Conclusion)

It has been demonstrated that the administration of NMN in infancy inhibits accumulation of white fat after weaning. The present invention leads to elucidation of physiological significance of NMN contained in breast milk, and is useful for healthy growth and development of many children.

### [Production Example of modified milk powder]

A modified milk powder can be obtained by adding NMN to a raw material so that the concentration in the final product (in the case of a modified milk powder used by dissolving NMN so as to be 5 to 17% (w/v)) is 0.1 to 100 mg/100 g, for example, 16.2 to 100 mg/100 g, dissolving NMN, mixing, and cleaning the mixture, then sterilizing, concentrating, homogenizing, and spray-drying the mixture, according to conventional methods.

### Industrial Applicability

According to the present invention, it is possible to provide a medicine or a food composition capable of improving exercise capacity of children in the future, and a method for producing a medicine or a food. According to the present invention, it is possible to provide a medicine or a food composition capable of reducing blood triglyceride levels in children in the future, and a method for producing a medicine or a food. According to the present invention, it is possible to provide a medicine or a food composition capable of reducing blood glucose levels in children in the future, and a method for producing a medicine or a food. According to the present invention, it is possible to provide a medicine or a food composition capable of inhibiting fat accumulation in children in the future, and a method for producing a medicine or a food. In addition, the present invention can be expected to realize improvement in nutrition, ensure a healthy life, and promote welfare of many children.

## Claims

1. A children's nutritional composition, comprising a precursor of nicotinamide adenine dinucleotide (NAD), for promotion of child development in any one selected from improvement in exercise capacity, reduction in blood triglycerides, reduction in blood glucose levels, and inhibition of fat accumulation.

2. The composition according to claim 1, wherein the composition is for improving exercise capacity, and the improvement in exercise capacity is any one selected from the group consisting of promotion of skeletal muscle development and improvement in activity.

3. The composition according to claim 1, wherein the composition is for improving exercise capacity, and the improvement in exercise capacity is measured by any one selected from the group consisting of improvement in muscle strength, increase in the amount of voluntary activity, and increase in moving speed.

4. The composition according to claim 1, wherein the promotion of child development is promotion of child development after weaning.

5. The composition according to claim 1, wherein the promotion of child development is promotion of child development after 3 years old.

6. The composition according to any one of claims 1 to 5, which is to be taken by an infant or toddler.

7. The composition according to any one of claims 1 to 5, which is to be taken by a child before weaning.

8. The composition according to any one of claims 1 to 5, wherein the composition is in any form selected from the group consisting of a modified milk powder, modified liquid milk, and a breast milk substitute food.

9. The composition according to any one of claims 1 to 5, wherein the precursor of NAD is nicotinamide mononucleotide (NMN).

10. The composition according to claim 9, wherein the concentration of NMN during intake is 0.05 µg/mL or more.

11. The composition according to claim 9, which is in a form of a modified milk powder and comprises 0.1 mg or more of NMN per 100 g.

12. A composition comprising a precursor of nicotinamide adenine dinucleotide (NAD), which is to be taken by a child before weaning for promoting child development after weaning.

13. The composition according to claim 12, wherein the promotion of child development is any one selected from improvement in exercise capacity, reduction in blood triglycerides, reduction in blood glucose levels, and inhibition of fat accumulation.
